# EUROPÄISCHE PATENTANMELDUNG

(11) **EP 2 216 072 A1**
(43) Veröffentlichungstag der Anmeldung: **11.08.2010**
(21) Anmeldenummer: 10150788.7
(22) Anmeldetag: 14.01.2010
(51) Int. Cl.: A61N 1/39, G01R 31/36

(54) **Verfahren zur Bestimmung einer Stromversorgungs-Zustandsgröße in einem aktiven medizinischen Implantat**

(30) Priorität: 09.02.2009 DE 102009000728
(71) Anmelder: Biotronik CRM Patent AG, 6341 Baar (CH)
(72) Erfinder: Dörr, Thomas, 12437, Berlin (DE); Schmidt, Rainer-Michael, 10435, Berlin (DE); Blachut, Peter, 13357, Berlin (DE)
(74) Vertreter: Lindner-Vogt, Karin L.

(57) **Zusammenfassung**

Verfahren zur Bestimmung einer Stromversorgungs-Zustandsgröße, insbesondere des Erhaltungszustandes einer Batterie oder eines Akkus, in einem aktiven medizinischen Implantat, wobei die Stromversorgung einer vorbestimmten Belastung ausgesetzt und ihre Ausgangsspannung zu mehreren Zeitpunkten während mindestens eines Zeitabschnitts der Belastungs-Phase erfasst und die Messwerte einem Vergleich mit entsprechenden Vergleichswert oder der aus den Messwerten gewonnene zeitliche Verlauf der Spannung einem Vergleich mit mindestens einem Vergleichs-Verlauf unterzogen wird, wobei das Vergleichsergebnis als kennzeichnend für die Zustandsgröße genommen wird.

## Beschreibung

Die Erfindung betrifft ein Verfahren zur Bestimmung einer Stromversorgungs-Zustandsgröße in einem aktiven medizinischen Implantat.

Die Funktion aktiver medizinischer Implantate, speziell implantierter medizinelektronischer Geräte, hängt von der Verfügbarkeit einer ausreichenden Betriebsenergie entscheidend ab. Da diese Geräte zumeist eine für den jeweiligen Patienten permanent oder im Notfall lebenswichtige Funktion haben, ist eine hinreichende Kenntnis des Zustandes der Stromversorgung, insbesondere des Erhaltungszustandes einer Batterie oder eines Akkus des Implantats, unabdingbar.

Die bislang bekannten Methoden, die Batteriezuverlässigkeit in ICD zu beurteilen, sind nur sehr bedingt geeignet, Batterieprobleme frühzeitig zu identifizieren und so ein Zuverlässigkeitsproblem des ICD zu vermeiden. Derzeit werden die Batteriespannungen, die minimale Batteriespannung während eines Ladevorganges und die Ladezeiten als Kriterien für die Batteriebeobachtung herangezogen.

Alle diese Messwerte sind durch Umgebungsfaktoren derart beeinflusst, dass eine spezifische Analyse des Batteriezustandes nur sehr eingeschränkt möglich ist. So wird die Ladezeit maßgeblich vom Formierungszustand der Schockkondensatoren beeinflusst. Die Batteriespannung ist abhängig von den aktuellen Betriebseinstellungen des ICD (mit oder ohne Stimulation etc.). Eine Früherkennung von Batterieproblemen ist mit den o. g. Methoden daher nicht möglich. Insbesondere ein sogenanntes Voltage Delay wird erst dann erkannt, wenn die Batterieleistung schon wesentlich und nicht reversibel reduziert ist.

Speziell bei SVO(Silber-Vanadium-Dioxid)-Batterien ist ein Voltage Delay typisch. Um diesen zu vermeiden, werden diese Batterien unspezifisch reformiert. Dies führt zu einem unnötigen Ladungsverlust.

Der Erfindung liegt daher die Aufgabe zugrunde, ein Verfahren sowie eine Anordnung zur zuverlässigen und hinsichtlich der Realisierungsbedingungen flexiblen Bestimmung einer wesentlichen Zustandsgröße der Stromversorgung eines aktiven medizinischen Implantats, insbesondere des erwähnten Erhaltungszustandes der Batterie oder des Akkus, anzugeben. Insbesondere soll eine Früherkennung von Implantat-Stromversorgungsproblemen - und hierbei insbesondere eines sogenannten Voltage Delay - möglich sein.

Diese Aufgabe wird in ihrem Verfahrensaspekt durch ein Verfahren mit den Merkmalen des Anspruchs 1 und in ihrem Vorrichtungsaspekt durch eine Anordnung mit den Merkmalen des Anspruchs 9 gelöst. Zweckmäßige Fortbildungen des Erfindungsgedankens sind Gegenstand der jeweiligen abhängigen Ansprüche.

Die Erfindung schließt den Gedanken ein, die Stromversorgung, speziell die als Energiequelle dienende Batterie oder den Akku, einer vorbestimmten Belastung auszusetzen und während mindestens eines Zeitabschnittes dieser Belastungs-Phase eine zustandsrelevante physikalische Messgröße zu erfassen. Die Erfassung kann kontinuierlich bzw. quasi-kontinuierlich oder zu mehreren definierten Zeitpunkten erfolgen. Insbesondere wird die Ausgangsspannung der Stromversorgung unter der definierten Belastung erfasst.

Des Weiteren gehört zur Erfindung, dass die gemäß dem Messprogramm aufgenommenen Messwerte bzw. der daraus abgeleitete zeitliche Verlauf der Messgröße einer Bewertung unterzogen werden, die sich im technischen Sinne insbesondere als Vergleichsvorgang mit entsprechenden (d. h. zu entsprechenden Zeitpunkten bei Vergleichsmessungen aufgenommenen) Vergleichswerten oder als Vergleich der Zeitabhängigkeit mit einer Vergleichs-Kurve darstellt. Derartige Vergleichsvorgänge stellen heute Standardprozeduren dar, und entsprechende Einzelwert-Diskriminatoren oder Mustervergleichseinheiten sind kommerziell erhältlich.

Neben der gegenüber singulären Messungen der Batteriespannung unter möglicherweise undefinierten Bedingungen erzielten höheren Aussagekraft und Verlässlichkeit bieten das vorgeschlagene Verfahren und die entsprechende Anordnung flexible Optionen hinsichtlich einer Ausführung der Zustandsgrößen-Bestimmung entweder direkt im Implantat oder durch Fernüberwachung desselben, bis hin zu einem komplexen Überwachungssystem für eine Vielzahl von in einem großen Territorium betriebenen Implantaten.

Besonders vorteilhaft ist die Realisierung des Verfahrens und der Vorrichtung bei einem implantierbaren Kardioverter/Defibrillator (ICD), bei dem das Leben des Patienten in höchstem Maße von der unbedingten Verfügbarkeit ausreichender Kardioversions- bzw. Defibrillationsenergie im Notfall abhängt. Weiterhin ist die Erfindung aber auch vorteilhaft bei Herzschrittmachern oder sonstigen implantierbaren Gewebestimulatoren oder implantierbaren Medikamentendosierpumpen etc. nutzbar.

In einer weiteren Ausführung der Erfindung ist vorgesehen, dass die Spannung während des Zeitabschnittes quasi kontinuierlich erfasst und der Zeitverlauf vollständig abgespeichert oder unmittelbar, insbesondere per Telemetrie, nach außerhalb des Implantates übertragen wird. Alternativ kann das Verfahren so ausgestaltet sein, dass die Spannung während des Zeitabschnittes mehrfach, insbesondere mindestens dreimal und bevorzugt mindestens fünfmal erfasst wird und hieraus charakteristische Punkte oder Abschnitte des Zeitverlaufes bestimmt und abgespeichert oder, insbesondere per Telemetrie nach außerhalb des Implantates übertragen werden.

Die Bestimmung der Zustandsgröße, speziell der Batterieparameter, erfolgt insbesondere vor und/oder während und/oder nach einer Kondensatorreformierung oder aber auch vor und/oder nach einem Ladevorgang auf Maximalenergie. Zwischen Beginn und Ende der Batteriebelastung erfolgen in einer weiteren Ausführung der Erfindung mindestens drei Spannungsmessungen. Zwischen Beginn und Ende der Batteriebelastung werden insbesondere mindestens die Anfangsspannung, die Endspannung und die Stetigkeit des Spannungsverlaufes zwischen Anfangs- und Endspannung erfasst. Speziell werden pro Messvorgang mindestens fünf Spannungswerte vor, während und nach der Batteriebelastung zu definierten Zeitpunkten erfasst und an das Fernüberwachungssystem übertragen.

Besonders aussagekräftig wird das vorgeschlagene Verfahren, wenn die erfassten Messwerte oder der zeitliche Verlauf einem Vergleich mit mehreren vorbestimmten Vergleichswerten oder zeitlichen Verläufen unterzogen und die einzelnen Vergleichsergebnisse einer statistischen Auswertung unterworfen werden, wobei das Ergebnis der statistischen Auswertung als kennzeichnend für die Zustandsgröße genommen wird. Hierbei können speziell als Vergleichswerte bzw. Vergleichs-Zeitabhängigkeiten solche herangezogen werden, die für vergleichbare Geräte mit vergleichbaren Energiequellen in unterschiedlich gutem Betriebs- bzw. Erhaltungszustand erfasst wurden. Es können auf dieser Grundlage um charakteristische Punkte bzw. zeitliche Verläufe des Messwertes (speziell der Spannung) adäquate Toleranzbereiche definiert werden. Außerhalb des Toleranzbereiches liegende Messwerte bzw. Zeitabhängigkeiten werden als Warnhinweis gewertet und können Anlass zu geeigneten Maßnahmen, von einem Nachladen bzw. Reformieren bis hin zu einem Batterie- bzw. Geräteaustausch, geben.

Im Hinblick auf die spezielle Problematik des Voltage-Delay bei bestimmten Batterietypen ist in einer weiteren Ausführung der Erfindung vorgesehen, dass Messpunkte der Spannung derart festgelegt werden, dass ein sägezahnartiger Abfall und Wiederanstieg der Spannung unmittelbar nach Beginn der Belastungs-Phase mit hinreichender zeitlicher Auflösung erfasst wird.

Anordnungs- bzw. Systemaspekte der Erfindung ergeben sich weitgehend aus den oben erläuterten Verfahrensaspekten, so dass diese hier nicht nochmals dargestellt werden sollen.

Es soll jedoch ausdrücklich darauf hingewiesen werden, dass mit Anordnungen der vorgeschlagenen Art ein Überwachungssystem zur Zustandsüberwachung der Stromversorgungen einer Vielzahl aktiver medizinischer Implantate aufgebaut werden kann. Diese umfasst neben einer Mehrzahl von solchen Anordnungen einen Überwachungsserver zur Erfassung und Speicherung der von den Auswertungseinheiten der Anordnungen in Zuordnung zu jeweils einem Implantat gelieferten Ausgangsgrößen und optional weiterer in den Auswertungseinheiten vorliegender Daten sowie zur Aufbereitung derselben zur zentralen Überwachung und optional Betriebssteuerung der zum System gehörenden Implantate und eine Mehrzahl von jeweils einer oder mehreren Auswertungseinheiten zugeordneten Relaisstationen zur Datenübertragung zwischen den Auswertungseinheiten und dem Überwachungsserver, insbesondere über ein Mobilfunknetz.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1 und 2: graphische Darstellungen des zeitlichen Verlaufs der Batteriespannung eines aktiven Implantats bei definierter Belastung,
- Fig. 3: eine schematische Darstellung wesentlicher Komponenten einer erfindungsgemäßen Anordnung in einem implantierbaren Defibrillator,
- Fig. 4: eine graphische Darstellung zu einer Durchführung des erfindungsgemäßen Verfahrens an einem weiteren Batteriespannungsverlauf,
- Fig. 5: eine schematische Darstellung zur Erläuterung des erfindungsgemäßen Verfahrens anhand des Batteriespannungsverlaufes nach Fig. 4,
- Fig. 6: eine schematische Darstellung eines erfindungsgemäßen Überwachungssystems und
- Fig. 7: eine schematische Darstellung einer weiteren Ausführungsform der erfindungsgemäßen Anordnung.

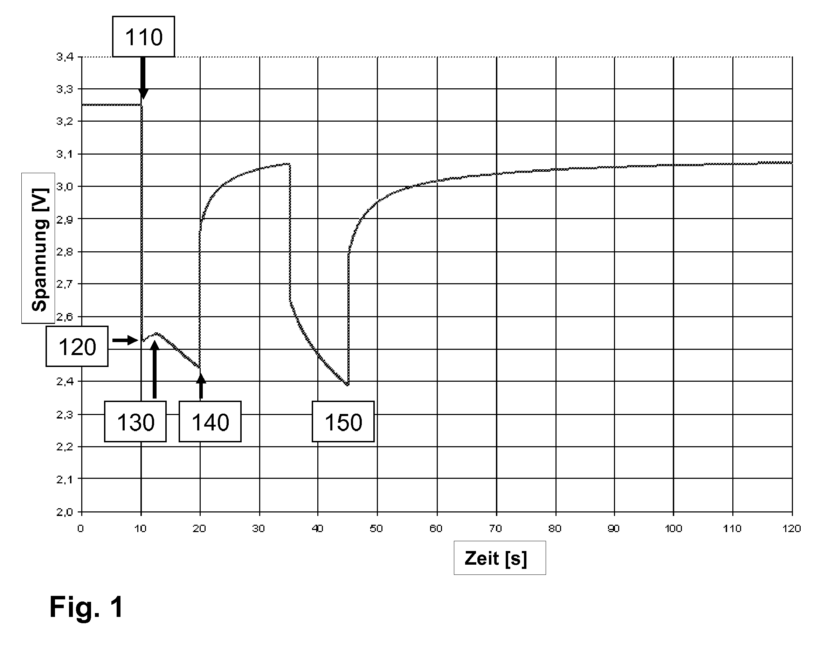

In Fig. 1 ist der Batteriespannungsverlauf einer ICD-Batterie bei zwei aufeinander folgenden Ladevorgängen dargestellt. Die Ruhespannung 110 bricht mit Beginn des Ladevorgangs, bedingt durch den Spannungsabfall am Innenwiderstand der Batterie, aber auch bedingt durch elektro-chemische Prozesse (dynamischer Innenwiderstand) auf einen Anfangswert ein (120). Während des ersten Ladevorgangs kommt es zunächst aufgrund chemischer Prozesse (Reformierung) innerhalb der Batterie zu einem leichten Spannungsanstieg 130. Dieser Spannungsanstieg ist Ausdruck eines leichten "Voltage Delay" und kann z. B. bei SVO-Batterien nach längerer Lagerung als noch normal bezeichnet werden. Die Endspannung liegt in diesem Fall deutlich unterhalb des Anfangswertes 120.

Der zweite Ladevorgang (Beginn bezeichnet mit 150) zeigt einen völlig normalen Spannungsverlauf mit einer stetigen Abnahme der Batteriespannung während der Batteriebelastung.

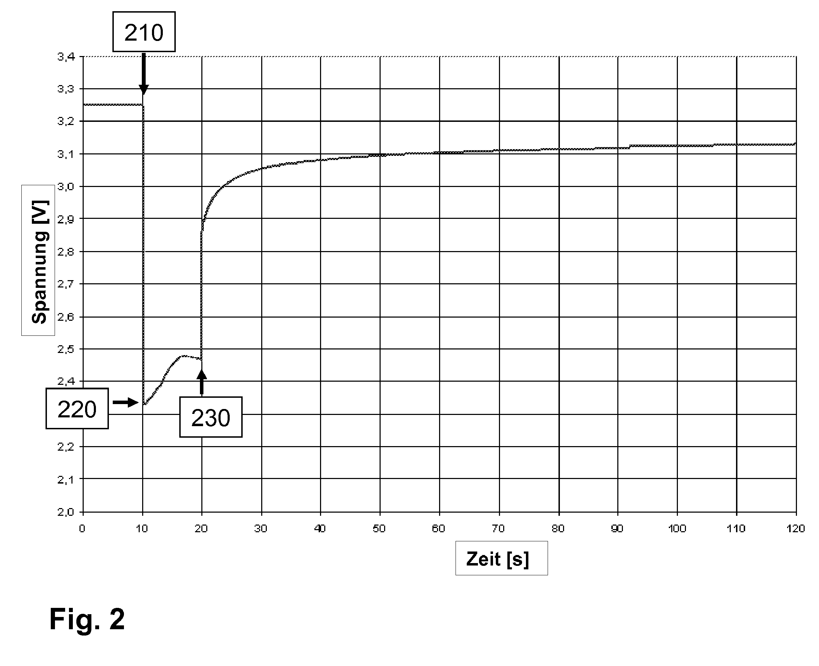

In Fig. 2 ist der Batteriespannungsverlauf einer ICD-Batterie mit einem ausgeprägten "Voltage Delay" dargestellt. Ausgehend von der unauffälligen Ruhespannung 210 der Batterie bricht die Spannung mit Beginn des Ladevorganges auf einen untypisch niedrigen Anfangswert 220 zusammen und steigt dann während des Ladevorganges an. Der Endwert 230 liegt in diesem Fall auf gleichem Niveau wie bei einem normalen Ladevorgang und deutlich oberhalb des Anfangswertes.

Der im Beispiel gezeigte Voltage Delay wäre noch nicht eindeutig an der Ladezeit erkennbar, da der Reformiereffekt der Batterie noch innerhalb des Ladevorgangs effektiv abgeschlossen wird. Ein solcher Spannungsverlauf ist jedoch ein eindeutiger Indikator für einen ausgeprägten Voltage Delay mit dem Hinweis auf einen erhöhten Reformierungsbedarf. In diesem Falle wäre es nötig, das Reformierungsintervall des ICD deutlich zu verkürzen, andernfalls wäre eine vorzeitige Batterieerschöpfung die Folge.

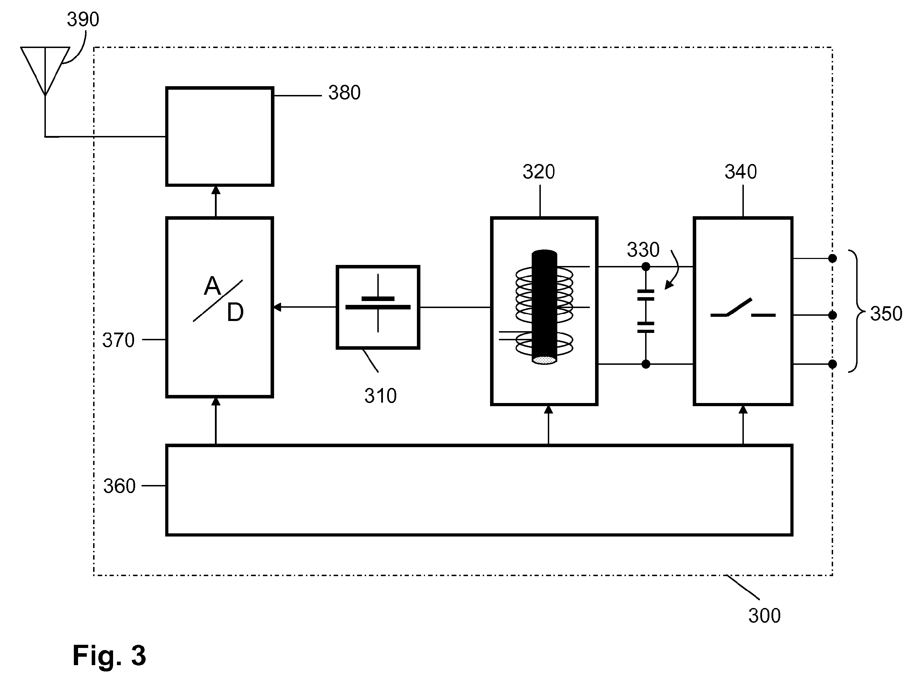

In Fig. 3 ist das Blockschaltbild eines implantierbaren Defibrillators (ICD) 300 dargestellt. Dieser hat eine Batterie 310, die u. a. die Ladeschaltung 320 für die Schockausgangsstufe 340 speist, die bei Bedarf die Schockenergie an die Defibrillationselektroden 350 weiterleitet. Die Steuerung der Ladevorgänge und die Schockabgabesteuerung übernimmt eine Steuereinheit 360. Diese Steuereinheit 360 steuert auch die mit der Batterie verbundene Batterieüberwachungseinheit 370, so dass die Messung der Batteriespannung zu definierten Zeitpunkten vor, während und nach dem Ladevorgang möglich ist.

Die Messwerte der Batterieüberwachungseinheit werden nach Abschluss der Messung der Telemetrieeinheit 380 zur Verfügung gestellt. Diese Telemetrieeinheit 380 arbeitet im MICS- oder GSM-Band und ist mit einer entsprechenden Antenne 390 zur Datenübertragung an ein Fernüberwachungssystem verbunden.

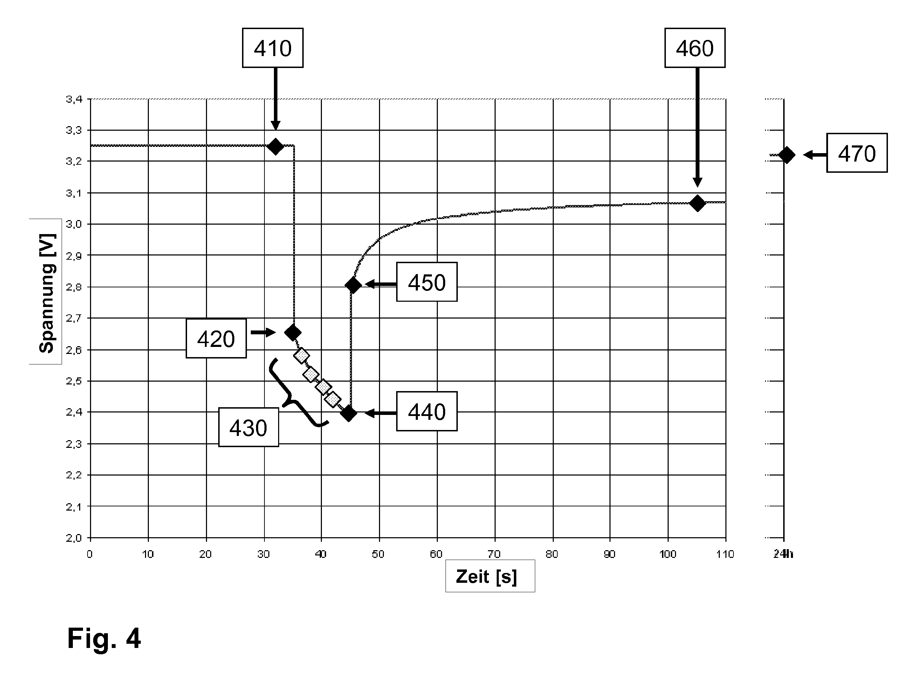

In Fig. 4 werden sinnvoll gewählte Zeitpunkte der Batteriespannungsmessung vor, während und nach einer Kondensatorreformierung dargestellt. Die in Fig. 3 dargestellte Steuereinheit 360 veranlasst die Batterieüberwachungseinheit 370 immer dann zu einer Serie von Batteriespannungsmessungen, wenn eine automatische Kondensatorreformierung ausgeführt wird. Die Ausführung der Messungen anlässlich einer Kondensatorreformierung hat den Vorteil, dass diese in regelmäßigen zeitlichen Abständen (z. B. alle 3 Monate) ausgeführt wird und immer ein definierter Ladevorgang (Start: Kondensatorspannung=0V; Ende Kondensatorspannung=Maximale Ladespannung) durchgeführt wird.

Eine erste Spannungsmessung erfolgt unmittelbar vor Beginn des Ladevorgangs 410, eine zweite Messung wird unmittelbar nach Einschalten der Ladeschaltung durchgeführt (420). Anschließend erfolgen alle 10-100 ms Messungen in einem konstanten zeitlichen Abstand (430), bis das Ladeende erreicht ist. Der letzte Messwert vor Ladeende (440) wird als solcher gekennzeichnet. Unmittelbar nach dem Abschalten der Ladeschaltung erfolgt eine weitere Messung (450). Die letzte Messung dieser Messreihe erfolgt 24h nach dem Ende der Kondensatorreformierung.

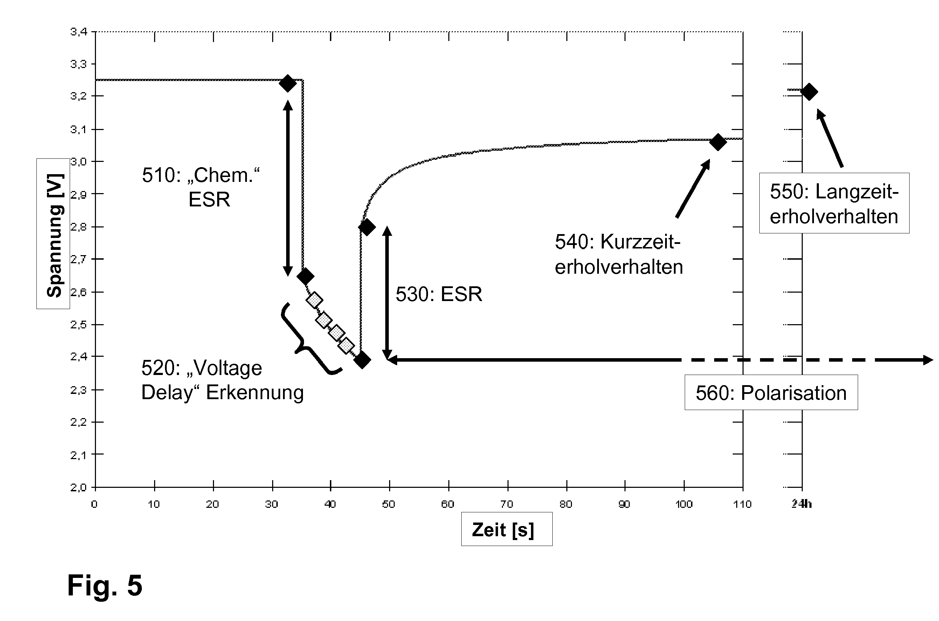

In Fig. 5 wird dargestellt, welche Batteriekenngrößen mit der erfindungsgemäßen Lösung bestimmt werden können. Die Differenz der Ruhespannung und der Anfangsspannung des Ladevorgangs geben einen Hinweis auf den sog. "chemischen" Innenwiderstand 510 der Batterie.

Die Aufzeichnung des Spannungsverlaufs während des Ladevorgangs 520 dient der Erkennung von beginnendem oder ausgeprägtem "Voltage Delay". Verlaufen die Messwerte stetig und liegt die Anfangsspannung über der Endspannung über der Endspannung ist kein Voltage Delay vorhanden. Liegt die Endspannung oberhalb der Anfangsspannung, so ist ein ausgeprägtes Voltage Delay vorhanden.

Die Spannungsdifferenz der Endspannung des Ladevorgangs und der unmittelbar nach Abschalten der Ladeschaltung gemessenen Spannung entspricht dem Innenwiderstand (530: ESR) der Batterie. Dessen Vergleich mit dem "Chem. ESR" 510 lässt zusätzlich Rückschlüsse auf die Stabilität des elektrochemischen Systems der Batterie zu.

Die eine Minute nach Ende des Ladevorgangs gemessene Batteriespannung ist Ausdruck für das Kurzzeiterholverhalten 540 der Batterie. Dieser Wert repräsentiert die Eigenschaften der Batterie, mehrere Ladevorgänge in kurzem Abstand (z. B. bei Schockserien) bedienen zu können.

Die nach 24 Stunden gemessene Batteriespannung 550 beschreibt das Langzeiterholverhalten der Batterie. Zu diesem Zeitpunkt ist die Erholung der Batteriespannung nach einem Ladevorgang abgeschlossen. Dieser Wert kann als zusätzliches Austauschkriterium für die Batterie genutzt werden.

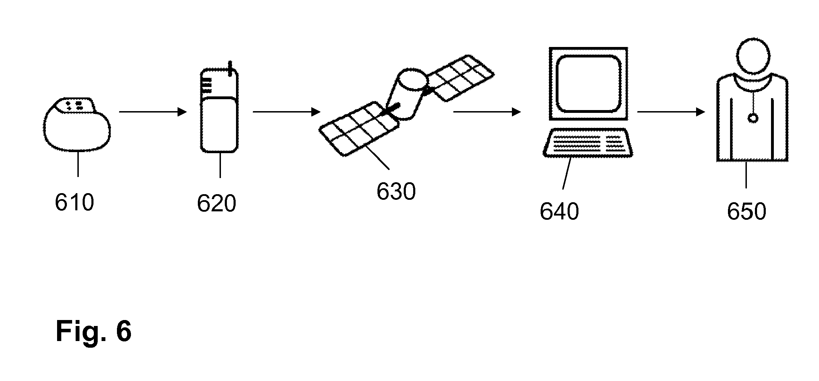

In Abbildung 6 ist schematisch ein die Batteriespannungswerte nutzendes Implantat-Überwachungssystem dargestellt. Ein Implantat 610 sendet in periodischen Abständen Nachrichten an eine Relaisstation 620. Zur Datenübertragung wird bevorzugt das MICS-Band und ein sog. Patientengerät oder alternativ das GSM-Band und eine GSM-Basisstation genutzt. Eine Relaisstation 620 überträgt die Daten anschließend via Netzwerk 630 an einen Fernüberwachungsserver 640 und stellt einem Arzt/Anwender 650 die Daten der Batteriediagnostik in aufbereiteter Form zur Verfügung. Die Aufbereitung erfolgt dabei bevorzugt in einer dem Anwender vertrauten Form wie z. B. einer Anzeige BOL, MOL, ERI/RRT oder EOS.

Die Darstellung in Fig. 6 ist stark vereinfacht; in der Praxis umfasst das dargestellte System selbstverständlich eine Vielzahl von Implantaten sowie auch eine Mehrzahl von zugeordneten Relaisstationen, und an den Überwachungsserver wird wiederum eine Mehrzahl von Computerarbeitsplätzen verschiedener Ärzte angeschlossen sein, die jeweils für die Betreuung eines oder mehrerer Patienten mit aktiven Implantaten zuständig sind und die gewonnenen und im System verarbeiteten Messwerte nutzen.

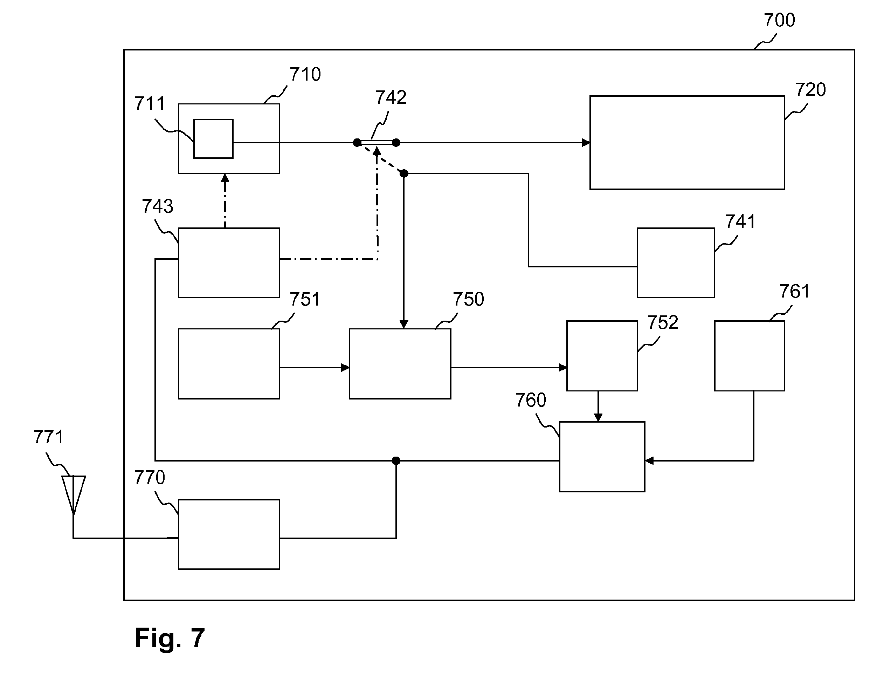

Fig. 7 zeigt im Zusammenhang mit der Erfindung wesentliche Komponenten eines weiteren aktiven medizinischen Implantats 700, und zwar zunächst eine Stromversorgung 710 mit einem Akku 711 als Energiequelle, zum Betrieb einer Funktionseinheit 720, etwa einem Stimulationsimpulsgenerator, einer Medikamentendosierpumpe o. ä. Außerdem ist bei dieser Ausführung eine spezielle Test-Last 741 vorgesehen, mit der der Akku 711 zur Ausführung einer Zustandstest-Messserie durch einen Schalter 742 verbunden wird, welcher seinerseits durch eine Stromversorgungs-Steuereinheit 743 betätigt wird.

Im mit der Last 741 verbundenen Schaltzustand der Stromversorgung 710 wird deren Ausgangsspannung über eine Spannungsmesseinrichtung 750 unter Steuerung durch eine Spannungsmess-Steuereinrichtung 751 nach einem dort gespeicherten Messprogramm in vorbestimmten Zeitabständen abgegriffen, und die erfassten Messwerte werden in einem Messwertspeicher 752 gespeichert. Die gespeicherten Messwerte werden einem Eingang einer Vergleichereinheit 760 zugeführt, deren anderer Eingang mit einem Vergleichswertspeicher 761 verbunden ist und von dort relevante Vergleichswerte (oder auch einen gespeicherten Vergleichs-Zeitverlauf) empfängt. Das Vergleichsergebnis kennzeichnende Ausgangswerte der Vergleichereinheit 760 werden zur Stromversorgungs-Steuereinheit 743 zurückgeführt, um dort als Eingangssignal zur Festlegung und Ausführung geeigneter, vom erfassten Zustand des Akkus abhängiger Vorgänge (soweit implantat-inter möglich) genutzt zu werden.

Zugleich wird das Vergleichsergebnis einer Telemetriestufe 770 mit einer Antenne 771 zur Übermittlung nach außerhalb des Implantats zugeführt, um dem betreuenden Arzt das Vergleichsergebnis und somit eine Aussage über den Zustand des Akkus 711 zu liefern. Der Arzt kann dann über etwa erforderliche Maßnahmen entscheiden, die eine äußere Einflussnahme auf das Implantat erfordern, etwa ein Nachladen oder ggf. einen Austausch des Akkus. Die Art der hierzu einsetzbaren externen Einrichtungen ist dem Fachmann ohne weiteres klar und bedarf daher hier keiner weiteren Erläuterung.

Die Ausführung der Erfindung ist nicht auf die oben beschriebenen Beispiele und hervorgehobenen Aspekte der Erfindung beschränkt, sondern ebenso in einer Vielzahl von Abwandlungen möglich, die im Rahmen fachgemäßen Handelns liegen.

## Patentansprüche

1. Verfahren zur Bestimmung einer Stromversorgungs-Zustandsgröße, insbesondere des Erhaltungszustandes einer Batterie oder eines Akkus, in einem aktiven medizinischen Implantat, wobei die Stromversorgung einer vorbestimmten Belastung ausgesetzt und ihre Ausgangsspannung zu mehreren Zeitpunkten während mindestens eines Zeitabschnitts der Belastungs-Phase erfasst und die Messwerte einem Vergleich mit entsprechenden Vergleichswert oder der aus den Messwerten gewonnene zeitliche Verlauf der Spannung einem Vergleich mit mindestens einem Vergleichs- Verlauf unterzogen wird, wobei das Vergleichsergebnis als kennzeichnend für die Zustandsgröße genommen wird.

2. Verfahren nach Anspruch 1, wobei die Spannung während des Zeitabschnittes quasi-kontinuierlich erfasst und der Zeitverlauf vollständig abgespeichert oder unmittelbar, insbesondere per Telemetrie, nach außerhalb des Implantates übertragen wird.

3. Verfahren nach Anspruch 1, wobei die Spannung während des Zeitabschnittes mehrfach, insbesondere mindestens dreimal und bevorzugt mindestens fünfmal erfasst wird und hieraus charakteristische Punkte oder Abschnitte des Zeitverlaufes bestimmt und abgespeichert oder, insbesondere per Telemetrie nach außerhalb des Implantates übertragen werden.

4. Verfahren nach einem der vorangehenden Ansprüche, wobei je ein Messwert der Ausgangsspannung nach Beginn und vor dem Ende der Belastungsphase erfasst wird und/oder Messwerte der Ausgangsspannung auch vor und/oder nach der Belastungs-Phase erfasst werden.

5. Verfahren nach einem der vorangehenden Ansprüche, wobei die Bestimmung der Zustandsgröße vor und/oder während und/oder nach einer Reformierung eines Energiespeicherkondensators des Implantates ausgeführt wird.

6. Verfahren nach einem der Ansprüche 1 bis 5, wobei die Bestimmung der Zustandsgröße vor und/oder während und/oder nach einem Vorgang des Ladens eines Energiespeicherkondensators des Implantates auf Maximalenergie ausgeführt wird.

7. Verfahren nach einem der vorangehenden Ansprüche, wobei die erfassten Messwerte oder der zeitliche Verlauf einem Vergleich mit mehreren vorbestimmten Vergleichswerten oder zeitlichen Verläufen unterzogen werden und die einzelnen Vergleichsergebnisse einer statistischen Auswertung unterworfen werden, wobei das Ergebnis der statistischen Auswertung als kennzeichnend für die Zustandsgröße genommen wird.

8. Verfahren nach einem der vorangehenden Ansprüche, wobei Messpunkte der Spannung derart festgelegt werden, dass ein sägezahnartiger Abfall und Wiederanstieg der Spannung unmittelbar nach Beginn der Belastungs-Phase mit hinreichender zeitlicher Auflösung erfasst wird.

9. Anordnung zur Durchführung des Verfahrens nach einem der vorangehenden Ansprüche, mit
einer Stromversorgungs-Steuereinheit zur Einleitung und Beendigung einer Belastungs-Phase einer Stromversorgung des Implantates mit einer vorbestimmten Last,
einer Spannungsmesseinrichtung zur Messung einer Ausgangsspannung der Stromversorgung,
einer Spannungsmess-Steuereinrichtung zur zeitlichen Steuerung der Spannungsmesseinrichtung derart, dass Messwerte zu vorbestimmten Zeitpunkten erfasst werden,
einem Vergleichswertspeicher zur Speicherung von Vergleichswerten der Spannung und/oder einem Vergleichszeitverlauf und
einer Vergleichereinheit zum Vergleich der zu bestimmten Zeitpunkten gewonnenen Messwerte mit entsprechenden Vergleichswerten und/oder eines aus den Messwerten zusammengesetzten zeitlichen Verlaufs mit dem Vergleichs-Zeitverlauf und zur Gewinnung einer das Vergleichsergebnis und somit die Zustandsgröße kennzeichnenden Ausgangsgröße.

10. Anordnung nach Anspruch 9, ausgebildet als Bestandteil eines aktiven medizinischen Implantates, insbesondere eines implantierbaren Kardioverters/Defibrillators.

11. Anordnung nach Anspruch 9, wobei die Stromversorgungs-Steuereinheit, die Spannungsmesseinrichtung und die Spannungsmess-Steuereinrichtung als Bestandteile eines aktiven medizinischen Implantates, insbesondere eines implantierbaren Kardioverters/Defibrillators, ausgebildet sind und dieses zusätzlich eine zur Übertragung der erfassten Messwerte nach außerhalb des Implantates ausgebildete Sendeeinrichtung, insbesondere Telemetrieeinrichtung, aufweist und wobei die übrigen Komponenten der Anordnung in einer Auswertungseinrichtung außerhalb des Implantates angeordnet sind, welche eine Empfangseinrichtung, insbesondere Telemetrieeinrichtung, zum Empfang der vom Implantat ausgesandten Messwerte aufweist.

12. Anordnung nach Anspruch 11, wobei die Auswertungseinrichtung zur Kommunikation mit einer Mehrzahl aktiver medizinischer Implantate und zur geordneten Speicherung und Vergleichsauswertung von Messwerten ausgebildet ist, die von diesen Implantaten gesendet werden.

13. Anordnung nach einem der Ansprüche 9 bis 12, wobei die Stromversorgungs-Steuereinheit eingangsseitig mit der Vergleichereinheit zur Aufnahme der Ausgangsgröße verbunden und derart ausgebildet ist, dass ein Lade- oder Reformier- oder ähnlicher Betriebsvorgang in der Stromversorgung in Abhängigkeit von der Ausgangsgröße ausgeführt wird.

14. Anordnung nach Anspruch 12 und 13, wobei der Vergleichereinheit in der Auswertungseinheit eine Statistikauswertungsstufe nachgeschaltet ist, welche zur Auswertung der von mehreren Implantaten gesandten Messwerte ausgebildet und deren Ausgang mit einer Sendeeinrichtung, insbesondere Telemetrieeinrichtung, der Auswertungseinheit zur Übermittlung eines des Ergebnis der statistischen Auswertung kennzeichnenden Ausgangssignals an eine Empfangseinrichtung des Implantates verbunden ist, und wobei die Empfangseinrichtung des Implantates mit einem Eingang der Stromversorgungs-Steuereinheit zur Übergabe des empfangenen Signals an diese verbunden ist.

15. Überwachungssystem zur Zustandsüberwachung der Stromversorgungen einer Mehrzahl aktiver medizinischer Implantate, mit einer Mehrzahl von Anordnungen nach einem der Ansprüche 11 bis 14, einem Überwachungsserver zur Erfassung und Speicherung der von den Auswertungseinheiten der Anordnungen in Zuordnung zu jeweils einem Implantat gelieferten Ausgangsgrößen und optional weiterer in den Auswertungseinheiten vorliegender Daten sowie zur Aufbereitung derselben zur zentralen Überwachung und optional Betriebssteuerung der zum System gehörenden Implantate und einer Mehrzahl von jeweils einer oder mehreren Auswertungseinheiten zugeordneten Relaisstationen zur Datenübertragung zwischen den Auswertungseinheiten und dem Überwachungsserver, insbesondere über ein Mobilfunknetz.
